# EUROPEAN PATENT SPECIFICATION

(11) **EP 0 927 544 B1**
(45) Date of publication and mention of the grant of the patent: **05.02.2003**
(21) Application number: 96946397.5
(22) Date of filing: 10.09.1996
(51) Int. Cl.: A61C 17/00, A46B 15/00, A46B 9/04, A61N 5/06

(54) **TOOTHBRUSH**
ZAHNBÜRSTE
BROSSE A DENTS

(43) Date of publication of application: 07.07.1999
(73) Proprietor: Altshuler, Grigory, Borisovich, St Petersburg, 196240 (RU)
(72) Inventor: Altshuler, Grigory, Borisovich, St Petersburg, 196240 (RU)
(74) Representative: Lange, Thomas, Dr.
(86) International application number: PCT/RU96/00257
(87) International publication number: WO 98/010711

(56) References cited:
- EP-A- 0 593 375
- WO-A-95/10243
- DE-U- 29 517 758
- US-A- 5 030 090

## Description

The invention relates to tooth brushes and can be used in dentistry for profilaxis and treatment of oral deseases.

It is known the tooth brush (PCT N90/0906, A61 N1/32, A46 B15/00, date of publication 23.08.90) comprising the electrode on the handle and the electrode with sharpened edges in the base of bristle. The electronic circuit providing the conditions of application of bipolar pulse train (especially with 50 Hz frequency) to oral tissues and LED indicating the operation of electronic circuit are mounted inside the brush handle.

The main disadvantage of the device above is that it is impossible to make the influence on teeth except the gum tissue.

Also a tooth brush is known which is the closest one from the technical point of view and can be accepted as a prototype (Japan N3-15883, A46 B15/00, A61 N5/06, D01 F8/04, date of publication 04.03.91) comprising the handle with built-in power supply, heater and head with bristle made of special thermoactive material radiating in far infrared range.

The main disadvantage of the prototype is the absence of radiation providing the profilaxis and treatment influence on teeth and gum.

In WO 95/10243 a further tooth brush for light treatment is disclosed. LED's are arranged in form of a linear array at the periphery of the bristle or within the bristle area. No reflection means are integrated in the brush head and further, the bristle is not made of a transparent material.

EP 0 593 375 A1 discloses a tooth brush, which is equipped with a brush head being made of two pieces of a transparent material. A plurality of reflecting coatings is integrated in the head. Again, the bristle is not transparent and thus decreases the intensity of the outputted radiation to the detriment of the medical treatment efficiency.

A prior art document, in which a bristle composed of transparent fibers is disclosed, is U.S. 5,030,090. In this document, the transparent fibers extend, as a fiber bundle, through the whole length of a brush base to the laser and include a bent portion in an intermediate region. Light is only transmitted via the fiber bundle and not through the brush base.

In the German Utility Model DE 295 17 758 U1 a tooth brush with a light source provided for illuminating the teeth and gums with irradiating light is disclosed. The front piece of the tooth brush is realized as a light transmitting guide and provided with an internal reflector at its end region. The reflector directs the irradiating light to an aperture situated in between the brushes.

It is an object of the present invention to provide a tooth brush for light irradiation treatment of the oral cavity

This object is solved by a tooth brush comprising a removable brush head with bristle, a handpiece with cavity, a light source, and a power supply, which is built-in the handpiece, wherein said light source is a source of radiation of optical range and brush head is made of transparent material. Further, said brush head is covered by a reflective or light scattering coating so that the most part of the radiation reaches the bristle, and said bristle is transparent.

Thus, the present invention consists of the creation of a tooth brush providing for profilaxis and treatment influence on tooth tissues and soft oral tissues.

Under the realization of the invention, the technical result consisting of application of radiation of optical range that provides the anti-inflammatory and caries protective influence on oral tissues as well as stimulates their regeneration.

The said light source may be represented by laser diode or light emitting diode.

The said light source may be represented by a filament lamp and the handpiece is made of color transparent material.

The said brush head may contain the light scattering materials.

The said brush head may contain the photoluminescent substances especially dyes.

It is well known the phisiotherapeutic influence of visible and near UV and IR radiations which at low doses make the biostimulating action on tissues. The most efficient is the laser radiation (A.S. Kryuk, V.A. Mostovnikov, I.V. Khokhlov, N.S. Serdyuchenko. Therapeutic efficiency of low-intensity laser radiation. Minsk: Science & Engineering 1986, V.E. Illarionov. The principles of laser therapy. M. Published by "RESPECT" of "INOTECH-Progress" Co.).

It is found out the antibacterial and anti-inflammatory action of UV (330-380 nm), blue (440-450 nm) and green (514-590 nm) radiation. Besides that the red (630-640 nm) and near IR (830-1300 nm) radiation provides also the profilaxis and caries treating influence.

The profilaxis influence against caries is provided by irradiation of odontoblasts and tooth pulp due to waveguide effect of light propagation in the enamele prisms and dentinal tubules (see G.B. Altshuler, V.N. Grisimov. "The effect of waveguide light propagation in human tooth". Doklady AN USSR, v. 310, N5, pp. 1245-1248, 1990; G.B. Altshuler, V.N. Grisimov. "New optical effects in the human hard tooth tissues". Proc. SPIE. Lasers and Medicine, v. 1353, pp 97-102, 1991).

While the gums are irradiated both the antiparadontosis action and penetration of light inside teeth take place. The radiation is most efficient in the combination with massage of gums because the pressuring of alive soft tissue causes the increase of its transparency (see G.A. Askaryan "The increasing of transmission of laser and other radiation through the soft turbid physical and biological media". Kvantovaya Electronika, v. 9, N7, 1982, pp. 1370-1383).

The usefull irradiation of a whole oral cavity is provided in the claimed tooth brush due to the presence of light source connected to the electrical power supply and transparent brush head which can be made of light scattering material.

The types of light sources are determined by the necessity of application of light with specified spectral parameters and dose that are useful for specified type of hard and soft oral tissues.

More intensive irradiation of teeth and gums are provided with tooth brush that has the transparent bristle and reflecting or back scattering coating of a brush head.

The photoluminescent substances especially the dyes being introduced into the brush head provide practically all the useful spectral range of oral cavity radiation under the influence of a single shortwave, for example, UV or blue light source.

The author supposes that the set of claims is the new one as well as the engineering satisfies the criterion of invention.

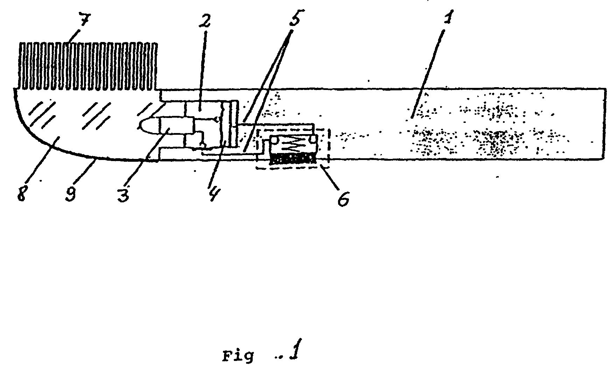

The subject of the invention is specified by the figures, where
fig. 1 shows the tooth brush which brush head has the reflecting coating and bristle is transparent.

The tooth brush (fig. 1) comprises the handpiece 1 with cavity 2, the radiation source 3 built-in the handpiece cavity and electrically connected to the power supply 4 through the contacts 5 of switcher 6. The bristle 7 is fixed on the transparent brush head 8.

When the filament lamp is used as a radiation source 3 the brush head 8 is made of color transparent material.

The brush head 8 is covered by reflecting coating 9 (fig. 1) as well as it may be made of light scattering material and covered by it. The bristle 7 is transparent.

The device operates in a following way. After the power supply of radiation source 3 is switched on by pressing the button of switcher 6 the radiation from the source 3 reaches the body of brush head 8 and gets in the oral cavity.

If the filament lamp is used as a radiation source 3 the spectral filter cutting the desirable spectral range should be applied because of the wide sprectral band of the source. The brush head made of the color (green, blue, or red) transparent material may play the role of such a filter.

If the bristle is made of the transparent material especially in that case when the brush head 8 is covered by reflecting coating 9 or when the radiation is scattered within the brush head the most part of radiation reaches the bristles, concentrates in them and then reaches the places of contact between bristle 7 and teeth or gum tissues.

Photoluminescent impurities, for example, dyes inside the brush head 8 provide the irradiation of oral cavity not only by radiation delivered from the radiation source 3 but also by radiation which spectral parameters are defined by Stokes law.

The wavelength of radiation emitted by the photoluminescent substances is always longer than the wavelength of radiation source 3. If there is a single UV or blue radiation source it allows to deliver to the oral cavity the other desirable spectral components of visible and infrared light.

The example of claimed device is the following: laser diodes are SDL-2380-S with 810 nm radiation wavelength and SDL-7430 with 675 nm radiation wavelength (see Product Catalog SDL "Semiconductor Diode Lasers" 1995), light emitting diodes are LEDS-5 and LEDS-3 (blue, green, red) (see Catalog "RS components", Vienna, 1995), the low-dimension power supply is VARTA chrom 547.

Thus taking into account the above, the claimed device allows to solve the problem of profilaxis and treatment influence on oral tissues.

## Claims

1. A tooth brush comprising:
a removable brush head (8) with bristle (7);
a handpiece (1) with cavity (2);
a light source (3); and
a power supply (4), which is built-in the handpiece (1), wherein said light source (3) is a source of radiation of optical range and brush head (8) is made of transparent material,
**characterized in that**
said brush head (8) is covered by a reflective or light scattering coating (9) so that the most part of the radiation reaches the bristle (7), and said bristle (7) is transparent.

2. The tooth brush as defined in claim 1, wherein said coating (9) is applied to the back surface of the brush head (8).

3. The tooth brush as defined in claim 1, wherein said light source (3) is a laser diode.

4. The tooth brush as defined in claim 1, wherein said light source (3) is a light emitting diode.

5. The tooth brush as defined in claim 1, wherein said light source (3) is a filament lamp and brush head is made of colour transparent material.

6. The tooth brush as defined in claim 1, wherein said brush head (8) contains light scattering materials.

7. The tooth brush as defined in claim 1, wherein said brush head (8) contains photoluminescent substances.

## Patentansprüche

1. Zahnbürste mit:
einem abnehmbaren Bürstenkopf (8) mit Borste (7);
einem Handteil (1) mit einem Hohlraum (2);
einer Lichtquelle (3); und
einer Leistungsversorgung (4), die in das Handteil eingebaut ist, wobei die Lichtquelle (3) eine Strahlungsquelle im optischen Bereich ist und der Bürstenkopf (8) aus einem transparenten Material besteht,
**dadurch gekennzeichnet,**
**dass** der Bürstenkopf (8) mit einer reflektierenden oder lichtstreuenden Schicht (9) bedeckt ist, so dass der überwiegende Teil der Strahlung die Borste (7) erreicht, und dass die Borste (7) transparent ist.

2. Zahnbürste nach Anspruch 1, wobei die Schicht (9) auf der rückseitigen Oberfläche des Bürstenkopfes angebracht ist.

3. Zahnbürste nach Anspruch 1, wobei die Lichtquelle (3) eine Laserdiode ist.

4. Zahnbürste nach Anspruch 1, wobei die Lichtquelle (3) eine lichtemittierende Diode ist.

5. Zahnbürste nach Anspruch 1, wobei die Lichtquelle (3) eine Glühlampe ist und der Bürstenkopf aus einem farbigen transparenten Material besteht.

6. Zahnbürste nach Anspruch 1, wobei der Bürstenkopf (8) lichtstreuende Materialien enthält.

7. Zahnbürste nach Anspruch 1, wobei der Bürstenkopf (8) photolumineszierende Substanzen enthält.

## Revendications

1. Brosse à dents comprenant :
une tête de brosse amovible (8) avec des poils (7) ;
une pièce à main (1) avec une cavité (2) ;
une source de lumière (3) ; et
une alimentation en puissance (4), intégrée dans la pièce à main (1), ladite source de lumière (3) étant une source de rayonnement dans une plage optique, et la tête de brosse (8) étant réalisée en matériau transparent,
**caractérisée en ce que**
ladite tête de brosse (8) est couverte par un revêtement réfléchissant ou diffusant la lumière (9) de sorte que la majeure partie du rayonnement atteint les poils (7), lesdits poils (7) étant transparents.

2. Brosse à dents selon la revendication 1, dans laquelle ledit revêtement (9) est appliqué à la surface postérieure de la tête de brosse (8).

3. Brosse à dents selon de la revendication 1, dans laquelle ladite source de lumière (3) est une diode laser.

4. Brosse à dents selon la revendication 1, dans laquelle la source de lumière (3) est une diode électroluminescente.

5. Brosse à dents selon la revendication 1, dans laquelle la source de lumière (3) est une lampe à filament, et la tête de brosse est réalisée en une matière transparente de couleur.

6. Brosse à dents selon la revendication 1, dans laquelle ladite tête de brosse (8) contient des matériaux qui diffusent la lumière.

7. Brosse à dents selon la revendication 1, dans laquelle la tête de brosse (8) contient des substances photoluminescentes.
